# EUROPEAN PATENT SPECIFICATION

(11) **EP 3 341 870 B1**
(45) Date of publication and mention of the grant of the patent: **12.10.2022**
(21) Application number: 16754491.5
(22) Date of filing: 19.08.2016
(51) Int. Cl.: G16H 50/70, G16H 50/20

(54) **SYSTEM AND METHODS FOR EXTRACTING INFILTRATE INFORMATION FROM IMAGING REPORTS FOR DISEASE DECISION SUPPORT APPLICATIONS**
SYSTEM UND METHODE ZUR EXTRAKTION VON INFILTRATINFORMATIONEN AUS BERICHTEN ZU BILDGEBENDEN VERFAHREN FÜR MEDIZINISCHE ENTSCHEIDUNGSUNTERSTÜTZUNGSANWENDUNGEN
SYSTÈME ET METHODES D'EXTRACTION DES INFORMATION D'INFILTRAT DES RAPPORTS D'IMAGERIE POUR LES APPLICATIONS D'AIDE À LA DÉCISION

(30) Priority: 26.08.2015 US 201562210023 P
(43) Date of publication of application: 04.07.2018
(73) Proprietor: Koninklijke Philips N.V., 5656 AG Eindhoven (NL)
(72) Inventor: CHIOFOLO, Caitlyn, Marie, 5656 AE Eindhoven (NL); CHBAT, Nicolas, Wadih, 5656 AE Eindhoven (NL); VAIRAVAN, Srinivasan, 5656 AE Eindhoven (NL)
(74) Representative: van Oudheusden-Perset, Laure E.
(86) International application number: PCT/EP2016/069696
(87) International publication number: WO 2017/032709

(56) References cited:
- Carol Friedman: "Semantic Text Parsing for Patient Records" In: "Medical Informatics", 1 January 2005 (2005-01-01), Kluwer Academic Publishers, Boston, XP055313515, ISBN: 978-0-387-24381-8 vol. 8, pages 423-448, DOI: 10.1007/0-387-25739-X_15, pages 430, 434-435, 438-442
- ANDREW S. WU ET AL: "Evaluation of Negation and Uncertainty Detection and its Impact on Precision and Recall in Search", JOURNAL OF DIGITAL IMAGING, vol. 24, no. 2, 10 November 2009 (2009-11-10), pages 234-242, XP055313517, SA ISSN: 0897-1889, DOI: 10.1007/s10278-009-9250-4
- Stefan Gindl: "Negation Detection in Automated Medical Applications A Survey", , 1 October 2006 (2006-10-01), XP055313850, Retrieved from the Internet: URL:http://publik.tuwien.ac.at/files/pub-i nf_4604.pdf [retrieved on 2016-10-28]

## Description

### Background

With the increasing trend toward building interoperable systems, data from disparate sources are aggregated, allowing for prevalent electronic use of previously underutilized clinical markers for disease, alone or in combination with more traditional clinical markers. Such previously underutilized diagnostic-related information can be extracted from radiology reports and combined with clinical markers of disease, such as fever, tachycardia, or low oxygen levels in blood, to evaluate a patient's health status. Radiology reports include results of a reading of an imaging exam for a patient, and may also include information regarding suggested follow-up and recommendations. Chest x-ray (CXR) reports, a type of radiology report, provide vital information for diagnosing clinical conditions, including for example, pneumonia, acute respiratory distress syndrome (ARDS), and cardiogenic pulmonary edema (CPE). Infiltrate information is a type of diagnostic-related information that may be extracted from a chest x-ray report. However, diagnostic-related information in a CXR report is frequently buried in the text of a report, which requires a skilled reader to understand and interpret. Furthermore, the report reader may introduce bias into the report interpretation, depending on his or her field of experience and level of expertise.

Electronic extraction of diagnostic-related information is difficult, as it is resource-intensive to code the diagnostic-related information in real-time and define a diagnostic-related information in real-time and define a standard set of keywords for reviewing a CXR report. Moreover, annotation of CXR reports is often not disease specific, and keyword searches are often insufficiently sensitive if the searches do not include a sufficient number of search terms. Keyword searches are frequently insufficiently specific when the searches do not include natural language processing considerations like the progression of a condition, or the negation of identified diagnostic-related information and keywords.

Friedmann, "Semantic Text Parsing for Patient Records" Medical Informatics, Kluwer Academic Publishers, vol. 8, p. 423-448, 2005 discloses that accessibility to a comprehensive variety of different types of structured patient data may be critical to improvement in the health care process, yet most patient information is in the form of narrative text. It is held that semantic methods are needed to interpret and map clinical information to a structured form so that the information will be accessible to other automated applications. Some semantic methods that map narrative patient information to a structured coded form are described.

Document Carol Friedman: "Semantic Text Parsing for Patient Records"In: "Medical Informatics", 1 January 2005 (2005-01-01), Kluwer Academic Publishers, Boston, XP055313515,ISBN: 978-0-387-24381-8, discloses a method and system for extracting information on infiltrates from an imaging report, where the imaging report is parsed and classified based on infiltrate related keywords, directional words indicating a location of the infiltrate and negation words.

### Summary

A computer-implemented method for extracting information on infiltrates from an imaging report, and applying the report infiltrate information to clinical guidelines and clinical support applications, comprising: receiving the imaging report; parsing the report for infiltrate-related keywords; classifying the report based on the infiltrate-related keywords and at least one of directional words indicating a location of the infiltrates, negation words, and words canceling the negation words; presenting the classified report on a dashboard for clinical decision-making; and applying infiltrate information from the classified report in a real-time clinical application for detecting and predicting acute illness. The real-time clinical application comprises applying the classified report to schedule application of clinical decision support, CDS, algorithms, wherein if infiltrate-related keywords are found in the report, then a CDS algorithm relevant to the imaging report is applied; and if no infiltrate-related keywords are found in the report, then a CDS algorithm requiring imaging report infiltrates is not applied.

A system for extracting information on infiltrates from an imaging report, and applying the report infiltrate information to clinical guidelines and clinical support applications, comprising: a non-transitory computer readable storage medium storing an executable program; a memory storing a keyword database, including infiltrate-related keywords, directional words, and negation words; and a processor executing the executable program to cause the processor to perform the method of the preceding paragraph.

A non-transitory computer-readable storage medium including a set of instructions executable by a processor, the set of instructions, when executed by the processor, causing the processor to perform operations, comprising: receiving an imaging report; parsing the report for infiltrate-related keywords; classifying the report based on the infiltrate-related keywords and at least one of directional words indicating a location of the infiltrates, negation words, and words canceling the negation words; presenting the classified report on a dashboard for clinical decision-making; and applying infiltrate information from the classified report in a real-time clinical application for detecting and predicting acute illness. The real-time clinical application comprises applying the classified report to schedule application of clinical decision support, CDS, algorithms, wherein if infiltrate-related keywords are found in the report, then a CDS algorithm relevant to the imaging report is applied; and if no infiltrate-related keywords are found in the report, then a CDS algorithm requiring imaging report infiltrates is not applied.

### Brief Description of the Drawings

Fig. 1 shows a schematic drawing of a system according to an exemplary embodiment.
Fig. 2 shows a flow diagram of a method for using classified infiltrate chest x-ray reports according to a first exemplary embodiment.
Fig. 3 shows a flow diagram of an exemplary method of extracting infiltrate information, from step 202 in Fig. 2.
Fig. 4 shows a flow diagram of an exemplary method of classifying chest x-ray reports by infiltrate pathology, from step 204 in Fig. 2.
Fig. 5 shows a flow diagram of an exemplary method of applying classified infiltrate reports to computerized clinical guidelines from steps 210-211, applying classified infiltrate reports to advanced clinical decision support applications from steps 212-213, and applying classified infiltrate reports to determine a schedule for applying clinical decision support algorithms from steps 214-219, all from Fig. 2.
Fig. 6 shows a flow diagram of an exemplary method of checking for the presence of a clinical condition in a health institution, or checking for compliance with medical policy, from step 220 in Fig. 2.
Fig. 7 shows an exemplary dashboard with an exemplary classified infiltrate report and patient medical information.

### Detailed Description

The exemplary embodiments may be further understood with reference to the following description and the appended drawings, wherein like elements are referred to with the same reference numerals. The exemplary embodiments relate to a system and methods for extracting infiltrate information from chest x-ray reports, classifying the reports, and applying infiltrate information from classified chest x-ray reports to a real-time clinical application for detecting and predicting acute illness. Although exemplary embodiments specifically describe chest x-rays, it will be understood by those of skill in the art that the system and method of the present disclosure may be used to extract infiltrate information from reports of any type of study or exam requiring a reading, classify the reports according to the infiltrate information, and employ classified reports within illness detection and prediction applications, within any of a variety of hospital settings.

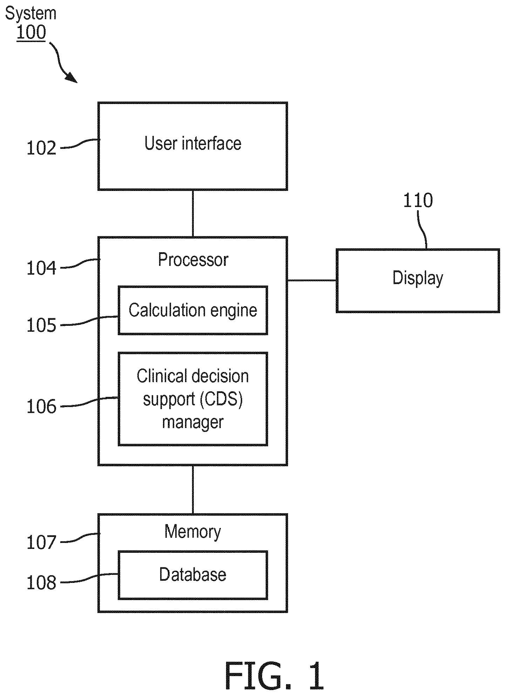

As shown in Fig. 1, a system 100, according to an exemplary embodiment of the present disclosure, extracts infiltrate information from chest x-ray reports, classifies the reports, and applies infiltrate information from classified chest x-ray reports for detecting and predicting acute illness. A chest x-ray report, for example, is a reading of results of a chest x-ray for the patient and may include relevant information regarding findings in the image along with follow-up suggestions and recommendations. The system 100 comprises a user interface 102, a processor 104, a memory 107, and a display 110.

The memory 107 includes a database 108, which stores prior and current chest x-ray reports for a patient, and a keyword database, including infiltrate-related keywords, directional words, negation words, and words canceling the negation words, etc. For example, exemplary databases may include electronic medical records (EMR), electronic health records (EHR), cloud-based databases, or data warehouses, etc. The processor 104 may be implemented with engines and modules including, for example, a calculation engine 105 and a clinical decision support (CDS) manager 106.

The calculation engine 105 extracts infiltrate information from a chest x-ray report and classifies the report based on presence, pathology, and location of infiltrates on the chest x-ray. The calculation engine 105 uses the classified reports to render user dashboards, select clinical decision support (CDS) algorithms, run computerized clinical guidelines, run advanced CDS algorithms, apply CDS algorithm selection rules for scheduling CDS algorithms, and check for compliance with medical policies. In an exemplary embodiment, the calculation engine 105 places the classified infiltrate report on a user dashboard or chart, along with medical test information on user interface 102 and display 110, to aid a medical care provider in clinical decision making. In another exemplary embodiment, the classified chest x-ray infiltrate report may be presented to a medical care provider via electronic health medical records on user interface 102 and display 110, to aid a medical care provider in clinical decision making.

In a further exemplary embodiment, the calculation engine 105 applies the classified infiltrate reports in computerized clinical guidelines, and runs the computerized guidelines to test for a specific disease. In another exemplary embodiment, the calculation engine 105 applies the classified infiltrate report in advanced clinical decision support (CDS) applications, to test for a specific disease. For example, the advanced CDS application may be a statistical model testing for a particular disease or clinical condition.

In another exemplary embodiment, the clinical decision support (CDS) manager 106 applies the classified reports to determine a schedule for running CDS algorithms, based on the infiltrate classification of the reports. In a further exemplary embodiment, the calculation engine 105 applies the classified infiltrate report to provide notification of disease or clinical conditions in a health institution. In another exemplary embodiment, the calculation engine 105 applies the classified infiltrate report to detect the presence of disease or a clinical condition in a health institution, or check for compliance with medical policies, including for example, hospital policies, safety protocols, or quality protocols.

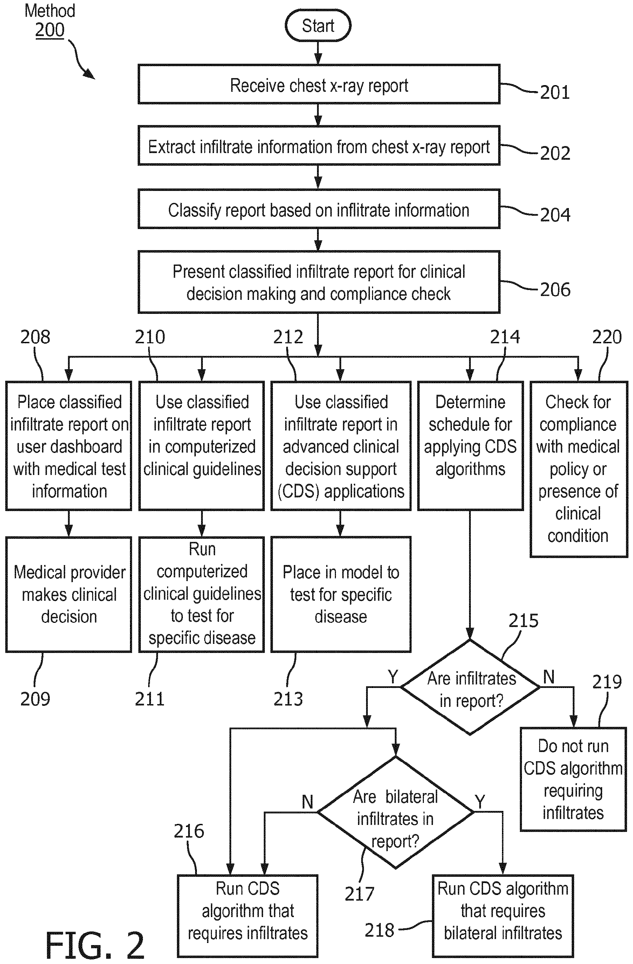

Fig. 2 shows an exemplary method 200 for extracting infiltrate information from chest x-ray reports, classifying the reports based on the infiltrate information, and applying the classified reports to a real-time clinical application for detecting disease, using system 100. The method 200 comprises steps for extracting infiltrate information from chest x-ray reports, processing the text of the report, classifying the reports, and applying the classified reports to real-time clinical applications, including one or more of running clinical guidelines, running CDS applications, scheduling CDS applications, detecting clinical conditions in a health institution, or running medical policy compliance checks, based on infiltrate information from the classified reports.

In step 201, the calculation engine 105 receives a chest x-ray report. In step 202, the calculation engine 105 extracts infiltrate information from the chest x-ray report. In step 204, the calculation engine 105 classifies the report based on the extracted infiltrate information. In step 206, the calculation engine 105 presents the classified infiltrate report to a medical care provider to aid in clinical decision making or determining compliance with hospital policy.

In step 208, the calculation engine 105 places the classified infiltrate report on a user dashboard or chart, along with medical test information, e.g. signs and symptoms of illness, patient demographics, patient vital signs, and biomarkers of disease or clinical conditions, etc. In step 209, the medical care provider makes a clinical decision based on the classified infiltrate report and the additional medical information on the dashboard. For example, while reviewing the user dashboard that presents the classified infiltrate report indicating the presence of unilateral infiltrates, elevated temperature (a fever), and presence of high microbe count of *K. pneumoniae* bacteria, a medical care provider may deduce the presence of pneumonia.

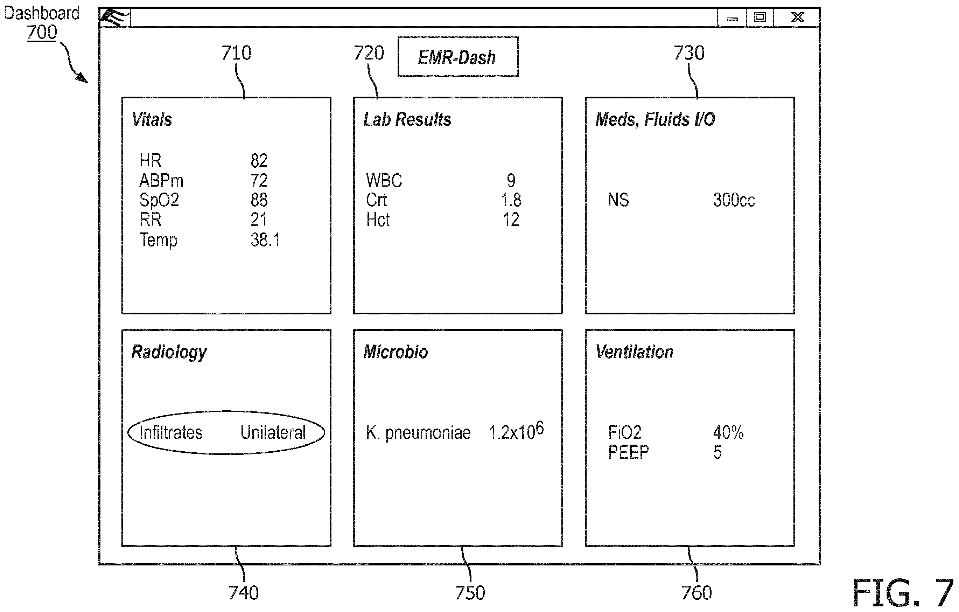

Fig. 7 shows, according to an exemplary embodiment of the present disclosure, a user dashboard 700 in the electronic medical records (EMR) presenting the classified infiltrate report 740 and the additional patient medical information. As depicted in Fig. 7, the additional medical information may include patient information, for example, patient vital sign information 710, lab results 720 on patient blood tests, information on medication 730, microbiology test results 750, and ventilation 760, etc.

Returning to Fig. 2, in step 210, the calculation engine 105 applies the classified infiltrate report in computerized clinical guidelines. In step 211, the calculation engine 105 runs the computerized clinical guideline to test for a specific disease.

In step 212, the calculation engine 105 applies the classified infiltrate report in advanced clinical decision support applications. In step 213, the classified infiltrate report is placed into a model that tests for a specific disease or clinical condition. In an exemplary embodiment, the advanced clinical decision support application may include an early detection model for acute respiratory distress syndrome (ARDS). For example, the model provides early detection of ARDS presence.

In step 214, the clinical decision support (CDS) manager 106 determines a schedule for running one or more CDS algorithms, based on the presence and classification of infiltrates on the CXR report. For example, the CDS manager 106 may optimize its performance and scheduling by automatically running only the relevant CDS algorithms. In step 215, the CDS manager 106 checks whether infiltrates are found in the report, using the CXR infiltrates algorithm. In step 219, when no infiltrates are present in the report, the CDS manager 106 does not run the CDS algorithm requiring CXR infiltrates as an input.

In step 217, the CDS manager 106 checks whether bilateral infiltrates are found in the report, using the CXR infiltrates algorithm. In step 216, when infiltrates are present in the report, or infiltrates are present but bilateral infiltrates are not present, the CDS manager 106 automatically runs only the relevant CDS algorithm, e.g. the algorithm that requires CXR infiltrates as an input. CDS algorithms that require CXR infiltrates as input include for example, algorithms for the detection of congestive heart failure (CHF), pneumonia, ARDS prediction, and tuberculosis.

In step 218, when bilateral infiltrates are present in the report, the CDS manager 106 automatically runs only the CDS algorithm that requires bilateral CXR infiltrates as an input. CDS algorithms that require bilateral CXR infiltrates as input including for example, ARDS guidelines and an algorithm for the ARDS-CPE exclusion model.

In step 220, the calculation engine 105 applies rules to check for clinical conditions in a health institution, or to check for compliance with medical policies, including, for example, hospital policies, or protocols for quality and safety reporting.

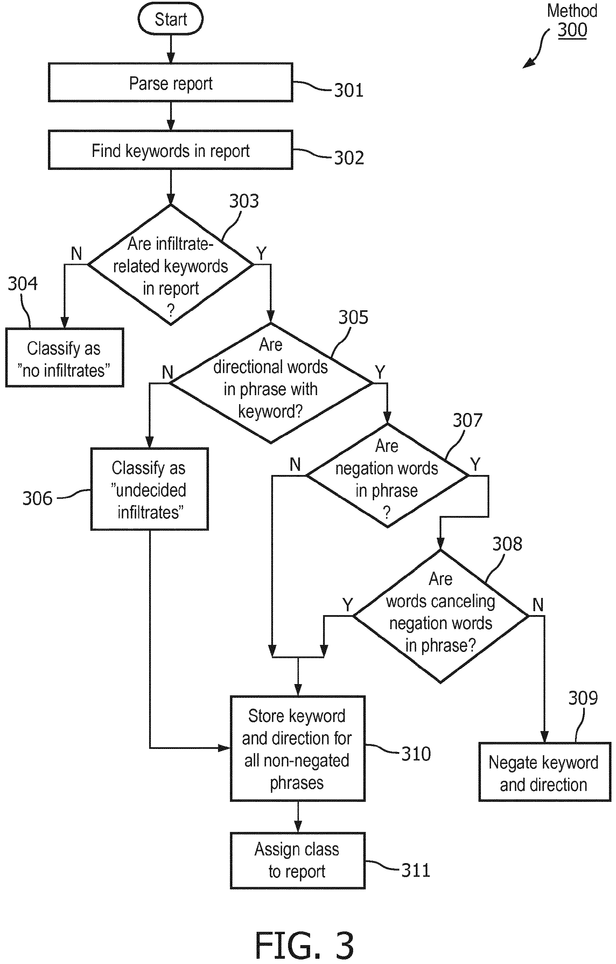

Fig. 3 shows an exemplary method for extracting infiltrate information from a chest x-ray (CXR) report, as depicted in step 202 in Fig. 2, depicted in further detail. The infiltrate information may include, for example, information on infiltrate pathology, e.g. location and presence of infiltrates. In step 301, the calculation engine 105 parses the CXR report, e.g. at the beginnings and ends of words and phrases.

In step 302, the calculation engine 105 detects keywords in the report relating to infiltrates, including, for example, infiltrates, consolidation, pneumonia, inflammation, infection, opacities, edema, multilobular, flooding, and densities, etc.

In step 303, the calculation engine 105 checks for the presence of infiltrate-related keywords. In step 304, when no infiltrate-related keywords are present in any phrase of the report, the report is classified as "no infiltrates." In step 305, when infiltrate-related keywords are present, the calculation engine 105 checks for the presence of directional words in the phrase containing the keyword. Exemplary directional words include, for example, bilateral, unilateral, bibasilar, both, right, and left, etc. In step 306, if there are no directional keywords in the phrase containing the keyword, the phrase is classified as "undecided infiltrates." This "undecided infiltrates" classification indicates that infiltrates are present in the phrase, and specifies that the particular location or side of the infiltrates is not clear. In step 310, the phrases classified as "undecided infiltrates" are stored with all non-negated phrases in the report. In step 311, the calculation engine 105 assigns a class to the report. If there are no other phrases containing keywords in the report, beside the phrases classified "undecided infiltrates," the calculation engine 105 classifies the report as "undecided infiltrates" in step 311. In another exemplary embodiment, rules are applied to further classify the phrase or report classified as "undecided infiltrates" as bilateral or unilateral.

In step 307, in a further exemplary embodiment, once the calculation engine 105 has detected directional words in the phrase with the keyword, the calculation engine checks for negation words in the phrase, including for example, no, not, negative, resolved, and without, etc. When no negation words are detected in the phrase with the keyword and directional words, in step 310, the keyword and direction from the directional words are not negated, e.g. the phrase has a keyword and directional words that are not ignored. Next, the non-negated keyword and direction for each phrase is stored, for all phrases with a non-negated keyword and direction in the report.

In step 308, once negation words are detected in the phrase, the calculation engine 105 checks for words that cancel the negation words in the phrase containing the keyword, including, for example, multiple negatives and adjectives, e.g. partially, slightly, not resolved, and no resolution, etc.

In step 309, when no words that cancel the negation words are detected in the phrase containing both the keyword and negation words, the keyword and direction are negated, e.g. ignored. For example, when a phrase contains a keyword "infiltrates," the directional word "left," negation words, and no words canceling the negation words, the keyword "infiltrates" and the direction of "left" are ignored.

In step 310, when words that cancel the negation words are detected in the phrase containing the keyword, directional words, and negation words, the negation words are canceled, and the keyword and direction from the directional words in the phrase are not negated, e.g. the keyword and directional words in the phrase are not ignored. Next, the non-negated keyword and direction for each phrase is stored, for all phrases with a non-negated keyword and direction in the report. In step 311, the calculation engine 105 assigns a class to the report, based on the keyword and directional words in the phrases of the report. Exemplary classifications include: "bilateral," "unilateral," or "undecided infiltrates," etc.

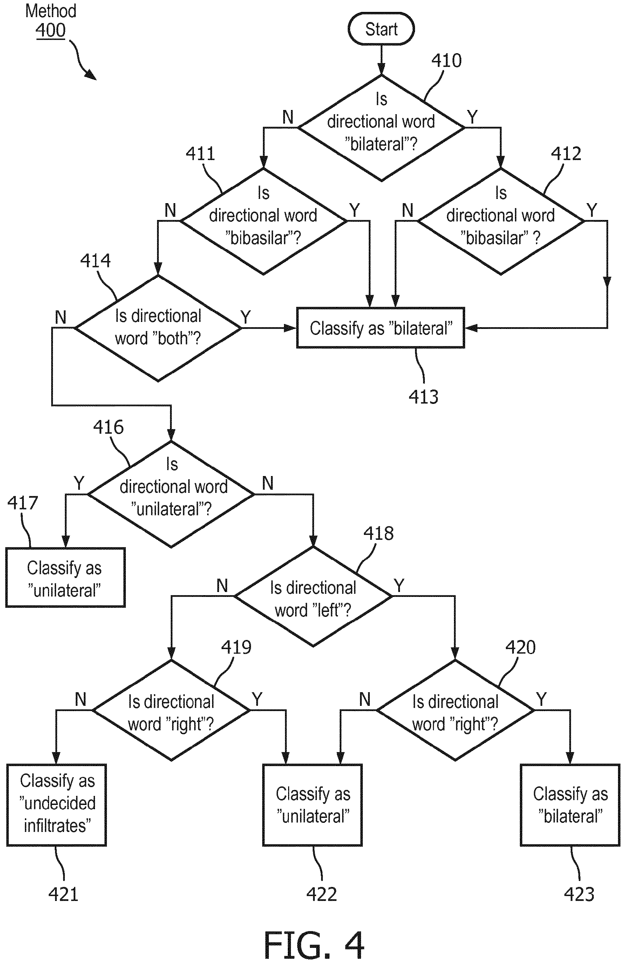

Fig. 4 shows an exemplary method for assigning a class to the report, as depicted in step 204 in Fig. 2, shown in further detail. In step 410, the calculation engine 105 checks if the directional word "bilateral" is present. In step 411, when the directional word "bilateral" is not present, the calculation engine checks if the directional word "bibasilar" is present. In step 412, when the directional word "bilateral" is present, the calculation engine checks if the directional word "bibasilar" is present. In step 414, when the directional words "bibasilar" and "bilateral" are both not present, the calculation engine 105 checks if the directional word "both" is present. In step 413, when at least one of the directional word "bilateral," "bibasilar," or "both" is present, or both directional words "bilateral" and "bibasilar" are present, the calculation engine 105 classifies the report as "bilateral." In step 416, when the directional word "bibasilar," "bilateral," and "both" are all not present, the calculation engine 105 checks if the directional word "unilateral" is present. In step 417, when the directional word "unilateral" is present, the calculation engine 105 classifies the report as "unilateral."

In step 418, when the directional word "unilateral" is not present, the calculation engine 105 determines whether the directional word "left" is in the phrase. In step 419, when the directional word "left" is not present, the calculation engine 105 checks if the directional word "right" is in the phrase. In step 420, when the directional word "left" is present, the calculation engine 105 checks if the directional word "right" is also present. In step 421, when the directional words "left" and "right" are both not present, the calculation engine 105 classifies the report as "undecided infiltrates," which indicates that infiltrates are present, but the location or side of the infiltrates is unclear. In step 422, when the directional word "left" is present and the word "right" is not present, or when the directional word "right" is present and the word "left" is not present, the calculation engine 105 classifies the report as "unilateral." In step 423, when the directional words "left" and "right" are both present, the calculation engine 105 classifies the report as "bilateral."

Table 1 below also presents exemplary rules determining the class assignment of the chest x-ray reports according to the presence of the directional words in the phrase.

**Table 1: Rules for Assigning a Class to Chest X-Ray Report**

| **Presence of Directional Word(s)** | **Assigned Class** |
|---|---|
| "Bilateral" | Bilateral |
| "Bibasilar" | Bilateral |
| "Both" | Bilateral |
| Both "Bilateral"; "Bibasilar" | Bilateral |
| Both "Left"; "Right" | Bilateral |
| "Unilateral" | Unilateral |
| "Right" and no "Left" | Unilateral |
| "Left" and no "Right" | Unilateral |

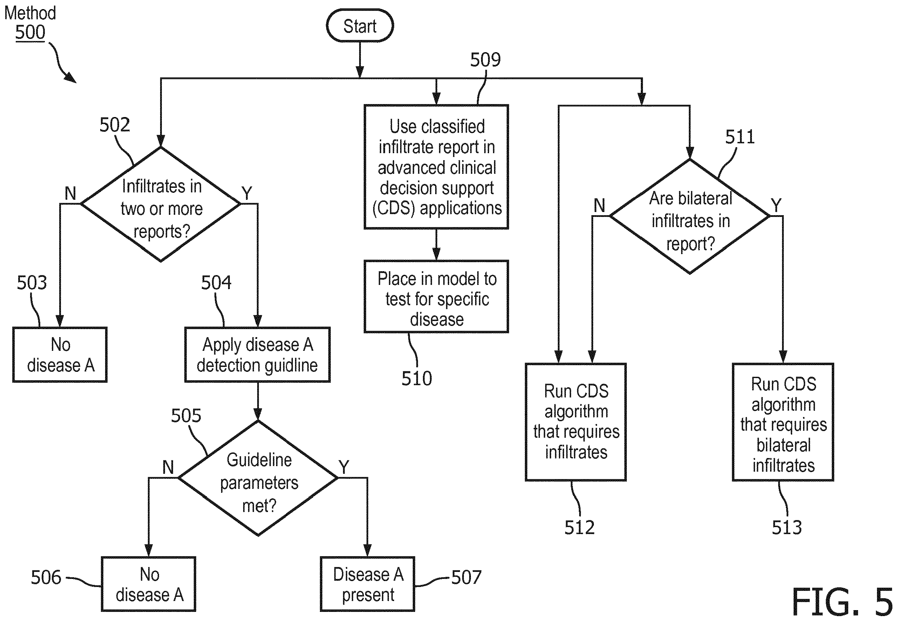

Fig. 5 shows a method 500 for applying classified infiltrate reports to computerized clinical guidelines and advanced clinical decision support applications, and determining a schedule for applying clinical decision support algorithms, from steps 210-211, 212-213, and 214-219 in Fig. 2, depicted in further detail.

In an exemplary embodiment, as depicted in steps 502-507, the calculation engine 105 applies the classified reports to run a computerized clinical guideline. For example, a computerized clinical guideline may be the guidelines for the detection of acute respiratory distress syndrome (ARDS). An exemplary computerized clinical guideline for detecting VAP is shown in steps 502-507. As another example, the classified infiltrate report could be used in the American-European Consensus Conference (AECC) and Berlin clinical guidelines for acute respiratory distress syndrome (ARDS), to test for the presence and severity of ARDS. The ARDS guidelines include for example, definitions of ARDS and are coded to the presence of bilateral infiltrates in the report, along with lab test results and ventilator settings. In another exemplary embodiment, the classified infiltrate reports could be used with clinical guidelines, e.g. detection of pneumonia, ventilator-associated pneumonia (VAP), cardiogenic pulmonary edema (CPE). In a further exemplary embodiment, the reports could be used for computerized clinical protocol-driven therapy recommendations.

An exemplary computerized clinical guideline for detecting VAP is shown in steps 502-507. In step 502, the calculation engine 105 checks whether infiltrates are present on two or more chest x-ray reports. In step 503, when infiltrates are not found in two or more reports, the ventilator-associated pneumonia (VAP) is not detected. In step 504, when infiltrates are present on two or more reports, the guideline for detecting a specific disease, for example, VAP, is applied. In step 505, the calculation engine 105 checks whether the parameters of the specific disease guideline are met. For example, in the VAP guideline, the calculation engine 105 checks for the presence of one of the following parameters: white blood cell count (WBC) of 1000 cells/ mm³ less than 4, or greater than or equal to 12, age over 70 years and Glasgow Coma Scale (GCS) less than 13, or temperature greater than 38 °C. In step 507, when the disease detection guideline parameters are satisfied, the disease is detected. For example, when any one of the above parameters is present, the calculation engine 105 checks for the presence of two of the following conditions: respiratory rate (RR) greater than 20, arterial oxygen partial pressure to fractional inspired oxygen ratio (PaO₂/ FiO₂) less than or equal to 240, and ventilator required. When two of these conditions are present, VAP is detected. In step 506, when the disease detection guideline parameters are not satisfied, the disease is not present. For example, when either the first set of VAP detection parameters (WBC, age, GCS, temperature) or the second set of parameters (RR, PaO₂/ FiO₂, ventilator requirement) is not satisfied, VAP is not detected.

In another exemplary embodiment, in step 509, the classified infiltrate reports may be used in advanced clinical decision support (CDS) applications, for example, in an early detection model for acute respiratory distress syndrome (ARDS). In this exemplary embodiment, in step 510, the classified infiltrate reports may be input into a model to test for a specific disease. For example, the CXR infiltrate classifications (no infiltrate, undecided, unilateral, bilateral) may each be coded with distinct numerical values, and the values for the classified reports may be input into a logistic regression or time series model with clinical information on patient vital signs, patient hospital status (admission, discharge, transfer) known as "ADT," and Intensive Care Unit (ICU) patient admission source. For example, this logistic regression model provides early detection of ARDS presence.

In a further exemplary embodiment, the classified infiltrate reports may be used to schedule and run relevant clinical decision support (CDS) algorithms. In step 511, when the CXR infiltrates algorithm determines that infiltrates are present in the report, the CDS manager 106 checks for the presence of bilateral infiltrates in the report. In step 512, when the CXR infiltrates algorithm determines that infiltrates are present in the report or that infiltrates are present, but bilateral infiltrates are not present, the CDS manager 106 runs only CDS algorithms relevant to the infiltrates in the report, for example, CDS algorithms that require infiltrates as an input, e.g. CDS algorithms for detecting congestive heart failure (CHF), pneumonia, ARDS prediction, and tuberculosis.

In step 513, when the CXR infiltrates algorithm determines that bilateral infiltrates are present, the CDS manager 106 runs only CDS algorithms relevant to the infiltrates in the report, for example, CDS algorithms that require bilateral infiltrates as an input, e.g. CDS algorithms for ARDS guidelines, or an ARDS-CPE exclusion model. In an exemplary embodiment, in the Intensive Care Unit (ICU), medical care providers may need to determine whether edema in the lung is present due to cardiogenic or non-cardiogenic causes. For example, when bilateral infiltrates are found in the report, the CDS manager 106 runs the ARDS-CPE exclusion model, which eliminates a cardiogenic cause for the edema, when the model indicates ARDS (a non-cardiogenic cause) is suspected to be present.

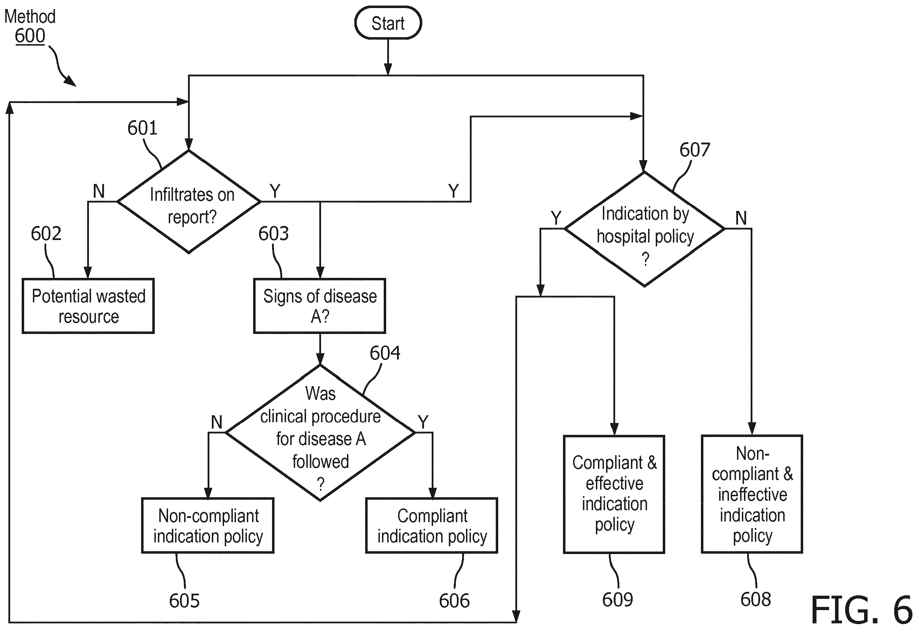

Fig. 6 shows a method 600 for reporting a clinical condition or disease in a health institution, or for checking for compliance with medical policies, including for example, hospital policy, or protocols for quality and safety reporting, as depicted in step 220 of Fig. 2, depicted in further detail. In an exemplary embodiment, in step 601, the calculation engine 105 checks for infiltrates on the chest x-ray report.

In step 603, when infiltrates are detected in the report, the calculation engine 105 checks for signs of a specific disease, for example, pneumonia. In an exemplary embodiment, the detection of infiltrates and signs of a specific clinical condition or disease could be used to report the presence of the clinical condition or disease in a health institution. In step 604, the calculation engine 105 determines whether the clinical procedure for the specific disease was followed. For example, in the case of a procedure for pneumonia, the calculation engine 105 first checks for ventilation, and then checks whether oral antiseptic has been ordered. That is, the calculation engine checks if any of the clinical procedure steps were not followed. In step 605, when the calculation engine determines that the clinical procedure for the specific disease was not followed, the calculation engine identifies the procedure as non-compliant with the hospital indication policy. For example, when one or both steps of the clinical procedure for pneumonia were not followed, the calculation engine identifies the procedure as non-compliant with hospital policy.

In step 606, when the clinical procedure for the specified disease was followed, the calculation engine 105 identifies the procedure as compliant with the hospital indication policy. For example, when all required steps of the procedure for pneumonia were followed, the procedure is identified as compliant with hospital policy.

In step 607, when the infiltrates are detected in the chest x-ray report in step 601, the calculation engine 105 then determines whether the detection had proper indications under the hospital policy. For example, the detection of infiltrates on a report may be a trigger for the calculation engine 105 to check whether the patient meets criteria for the hospital indication for chest x-ray policy, e.g. the calculation engine 105 runs a set of rules to see whether the patient meets necessary criteria that would have required a doctor to order a chest x-ray. As an example of a proper indication under the hospital policy, under the hospital's indication policy for chest x-rays, suspected pneumonia may be a valid criterion for ordering chest x-rays.

When the infiltrates are detected in the chest x-ray report in step 601 and the detected infiltrates are confirmed as having proper indications under the hospital policy in step 607, in step 609, the calculation engine 105 identifies the procedure as compliant with the hospital indication policy, a good use of resources, and an effective indication policy. Returning to the above example, the identification of infiltrates in a report may be the hospital indication requiring a doctor to order a chest x-ray, under the hospital indication policy. Thus, for example, when infiltrates are detected in a chest x-ray report in step 601, in step 607, it is determined that the use of chest x-rays complies with the hospital indication procedure for pneumonia, for ordering chest x-rays when infiltrates are detected in a report. In this example, when the physician orders a chest x-ray in compliance with hospital policy (e.g., patient had suspected pneumonia), and the patient's chest x-ray has infiltrates, then the hospital policy was effective in recommending the chest x-ray order. For example, quality officers at the institution may use the method to continuously monitor the effectiveness of hospital policies over time, and amend the policies as necessary, to improve the clinical workflow and quality of care provided to patients at the institution.

In step 608, once the calculation engine 105 determines that the detection of infiltrates did not have proper indications for performing a specific procedure under the hospital policy in step 607, the calculation engine identifies the procedure as non-compliant with the hospital indication policy, where the procedure may signal ineffective hospital indication for ordering the procedure. For example, when the positive detection of infiltrates is not a valid criterion for the hospital indication policy of using chest x-rays, e.g., in checking for asthma, the use of the chest x-ray is identified as non-compliant with hospital indication policy, and ineffective hospital indication for ordering the x-ray for asthma. In this example, the hospital x-ray order indication policy may be ineffective because the asthma cases show detected infiltrates, but the asthma cases are not covered by the current chest x-ray order indication policy, which may signal a need to change the chest x-ray order indication policy. Further in this example, when the physician orders a chest x-ray out of compliance with hospital policy (e.g., patient had asthma, but did not have suspected pneumonia), and the patient's chest x-ray had infiltrates, then the hospital policy for ordering chest x-rays should be corrected, if this scenario of detected infiltrates that is not compliant with hospital chest x-ray ordering policy is found for many patients' chest x-rays in the institution.

In another exemplary embodiment, once the calculation engine 105 identifies the procedure as compliant with the hospital indication policy in step 607, the calculation engine checks for the presence of infiltrates in the report in step 601. In step 602, when no infiltrates are detected despite a proper indication under hospital policy, the calculation engine 105 identifies a potential wasted medical resource, signaling an ineffective indication policy for ordering the chest x-ray. For example, after the calculation engine 105 determines the chest x-ray report results are compliant with the hospital policy indication in step 607 for requiring a doctor to order a chest x-ray, in step 601, the absence of detected infiltrates in the report is identified. As an example, the calculation engine 105 may determine in step 602 that the ordered chest x-ray is a potential wasted medical resource, where the chest x-ray may not have been required, for a lower-severity lung condition where infiltrates are not detected.

In a further exemplary embodiment of the invention, the calculation engine 105 may extract information on alternate risk factors, such as cardiac anomalies, for example, from the chest x-ray reports, to input into other predictive algorithms.

Those of skill in the art will understand that the above-described exemplary embodiments may be implemented in any number of matters, including, as a software module, as a combination of hardware and software, etc. For example, the exemplary method 200, exemplary method 300, exemplary method 400, exemplary method 500, exemplary method 600, calculation engine 105, and clinical decision support (CDS) manager 106 may be programs containing lines of code that, when compiled, may be executed by a processor.

Those skilled in the art will understand that the engines 105-106 may be implemented by the processor 104 as, for example, lines of code that are executed by the processor 104, as firmware executed by the processor 104, as a function of the processor 104 being an application specific integrated circuit (ASIC), etc.

## Claims

1. A computer-implemented method for extracting information on infiltrates from an imaging report, and applying the report infiltrate information to clinical guidelines and clinical support applications, comprising:
receiving the imaging report;
parsing the report for infiltrate-related keywords;
classifying the report;
presenting the classified report on a dashboard for clinical decision-making; and
applying infiltrate information from the classified report in a real-time clinical application for detecting and predicting acute illness;
**characterized in that**
the report is classified based on the infiltrate-related keywords and at least one of directional words indicating a location of the infiltrates, negation words, and words canceling the negation words; and **in that**
the real-time clinical application comprises applying the classified report to schedule application of clinical decision support, CDS, algorithms;
wherein:
if infiltrate-related keywords are found in the report, then a CDS algorithm relevant to the imaging report is applied: and
if no infiltrate-related keywords are found in the report, then a CDS algorithm requiring imaging report infiltrates is not applied.

2. The computer-implemented method of claim 1, wherein the imaging report comprises a chest x-ray report.

3. The computer-implemented method of claim 1, wherein the directional words comprise at least one of: bilateral, unilateral, bibasilar, right, left, both.

4. The computer-implemented method of claim 1, wherein the negation words comprise at least one of: no, not, negative, resolved, without, and wherein the words canceling the negation words comprise either: multiple negatives or
at least one adjective of: partially, slightly, not resolved, and no resolution.

5. The computer-implemented method of claim 1, wherein the infiltrate-related words comprise at least one of: infiltrates, consolidation, pneumonia, inflammation, infection, opacities, edema, multilobular, flooding, and densities.

6. The computer-implemented method of claim 1, wherein classifying the report further comprises:
detecting the infiltrate-related keywords in the report;
searching for the directional words in a phrase containing the detected infiltrate-related keywords;
searching for the negation words in the phrase;
searching for the words canceling the negation words in the phrase;
negating the infiltrate-related keywords and the directional words, when the phrase contains the negation words and does not contain the words canceling the negation words;
storing the infiltrate-related keywords and the directional words, for each phrase containing the negation words and not containing the words canceling the negation words; and
assigning a class to the report based on logical rules on a pathology and the location of the infiltrates.

7. The computer-implemented method of claim 6, wherein the logical rules comprise:
classifying the report as bilateral if the directional words include at least one of bilateral, bibasilar, or both; or the directional words include both left and right; and
classifying the report as unilateral if
the directional words include unilateral;
the directional words include right and not left; or
the directional words include left and not right.

8. The computer-implemented method of claim 6, wherein the logical rules comprise:
classifying the report as "no infiltrates," if the infiltrate-related keywords are not detected; and
classifying the report as "undecided infiltrates," if the infiltrate-detected keywords are detected in the phrase, but the phrase does not include any directional words.

9. The computer-imptemented method of claim 1, wherein the real-time clinical application comprises at least one of:
applying the classified report to computerized clinical guidelines for detecting a disease; and
applying the classified report to determine computerized clinical protocol therapy recommendations.

10. The computer-implemented method of claim 1, wherein the real-time clinical application comprises:
applying the classified report to advanced clinical decision support guidelines for detecting a disease, wherein applying the classified report to the advanced clinical decision support guidelines further comprises:
assigning a numerical value to each infiltrate
classification; and inputting the numerical values into a model for detecting a disease.

11. The computer-implemented method of claim 1, wherein the real-time clinical application comprises:
applying the classified report to provide notification of a disease or a clinical condition in a health institution.

12. The computer-implemented method of claim 1, wherein the real-time clinical application comprises:
applying the classified report to check compliance with at least one of hospital policy, hospital quality protocol, or hospital safety protocol; or
applying the classified report to monitor the effectiveness of the hospital policy.

13. A system for extracting information on infiltrates from an imaging report, and applying the report infiltrate information to clinical guidelines and clinical support applications, comprising:
a non-transitory computer readable storage medium storing an executable program;
a memory storing a keyword database, including infiltrate-related keywords, directional words, and negation words; and
a processor executing the executable program to cause the processor to perform a method according to any of the preceding claims.

14. The system of claim 13, wherein the real-time clinical application comprises:
applying the classified report to computerized clinical guidelines for detecting a disease.

15. A non-transitory computer-readable storage medium including a set of instructions executable by a processor, the set of instructions, when executed by the processor, causing the processor to perform operations, comprising:
receiving an imaging report;
parsing the report for infiltrate-related keywords;
classifying the report;
presenting the classified report on a dashboard for clinical decision-making; and
applying infiltrate information from the classified report in a real-time clinical application for detecting and predicting an acute illness;
**characterized in that**
the report is classified based on the infiltrate-related keywords and at least one of directional words indicating a location of the infiltrates, negation words, and words canceling the negation words; and **in that**
the real-time clinical application comprises applying the classified report to schedule application of clinical decision support, CDS, algorithms;
wherein:
if infiltrate-related keywords are found in the report, then a CDS algorithm relevant to the imaging report is applied: and
if no infiltrate-related keywords are found in the report, then a CDS algorithm requiring imaging report infiltrates is not applied.

## Patentansprüche

1. Computerimplementiertes Verfahren zum Extrahieren von Informationen über Infiltrate aus einem Bildgebungsbericht und zum Anwenden der Infiltrat-Informationen aus dem Bericht für klinische Richtlinien und klinische Unterstützungsanwendungen, das Folgendes umfasst: Empfangen des Bildgebungsberichts; Analysieren des Berichts nach Infiltrati-bezogenen Schlüsselwörtern; Klassifizieren des Berichts; Darstellen des klassifizierten Berichts auf einem Dashboard für die klinische Entscheidungsfindung; und Anwenden von Infiltratnformationen aus dem klassifizierten Bericht in einer klinischen Echtzeitanwendung zum Erkennen und Vorhersagen einer akuten Erkrankung; **dadurch gekennzeichnet, dass** der Bericht auf der Grundlage der Infiltrat-bezogenen Schlüsselwörter und mindestens eines von Richtungswörtern, die einen Ort der Infiltrate angeben, Verneinungswörtern und Wörtern, die die Verneinungswörter aufheben, klassifiziert wird; und dadurch, dass die klinische Echtzeitanwendung das Anwenden des klassifizierten Berichts auf die Zeitplananwendung von Algorithmen zur klinischen Entscheidungsunterstützung, CDS, umfasst: wobei:
wenn Infiltrat-bezogene Schlüsselwörter im Bericht gefunden werden, wird ein für den Bildgebungsbericht relevanter CDS-Algorithmus angewandt; und für den Fall, dass keine Infiltrat-bezogenen Schlüsselwörter im Bericht gefunden werden, wird kein CDS-Algorithmus angewandt, der Infiltrate im Bildgebungsbericht erfordert.

2. Das computerimplementierte Verfahren nach Anspruch 1, wobei der Bildgebungsbericht einen Röntgenbericht der Brust umfasst. Röntgenbericht umfasst.

3. Computerimplementiertes Verfahren nach Anspruch 1, wobei die Richtungswörter mindestens eines der folgenden umfassen: bilateral, unilateral, bibasilar, rechts, links, beides.

4. Computerimplementiertes Verfahren nach Anspruch 1, wobei die Verneinungswörter mindestens eines der folgenden Wörter umfassen: nein, nicht, negativ, aufgelöst, ohne, und wobei die Wörter, die die Verneinungswörter aufheben, entweder mehrere Negative oder mindestens ein Adjektiv der folgenden Wörter umfassen: teilweise, geringfügig, nicht aufgelöst und keine Auflösung.

5. Computerimplementiertes Verfahren nach Anspruch 1, wobei die Infiltrat-bezogenen Wörter mindestens eines der folgenden umfassen: Infiltrate, Konsolidierung, Lungenentzündung, Entzündung, Infektion, Trübungen, Ödeme, multilobulär, Überflutung und Dichten.

6. Computerimplementiertes Verfahren nach Anspruch 1, wobei das Klassifizieren des Berichts ferner Folgendes umfasst:
Erkennen der Infiltrat-bezogenen Schlüsselwörter in dem Bericht; Suchen nach den Richtungswörtern in einer Phrase, die die erkannten Infiltrat-bezogenen Schlüsselwörter enthält;
Suche nach den Verneinungswörtern in der Phrase; Suche nach den Wörtern, die die Verneinungswörter in der Phrase aufheben; Verneinung der Infiltrat-bezogenen Schlüsselwörter und der Richtungswörter, wenn die Phrase die Verneinungswörter enthält und nicht die Wörter, die die Verneinungswörter aufheben;
Speichern der Infiltrat-bezogenen Schlüsselwörter und der Richtungswörter für jede Phrase, die die Verneinungswörter enthält und nicht die Wörter, die die Verneinungswörter aufheben; und
Zuweisung einer Klasse an den Bericht auf der Grundlage logischer Regeln für eine Pathologie und die Lage der Infiltrate,

7. Computerimplementiertes Verfahren nach Anspruch 6, wobei die logischen Regeln Folgendes umfassen: Klassifizieren des Berichts als bilateral, wenn die Richtungswörter mindestens eines von bilateral, bibasilar oder beides enthalten; oder die Richtungswörter sowohl links als auch rechts enthalten; und Klassifizieren des Berichts als unilateral, wenn die Richtungswörter unilateral enthalten; die Richtungswörter rechts und nicht links enthalten; oder die Richtungswörter links und nicht rechts enthalten.

8. Computerimplementiertes Verfahren nach Anspruch 6, wobei die logischen Regeln Folgendes umfassen:
Klassifizieren des Berichts als " kein Infiltrat", wenn die Infiltrat-bezogenen Schlüsselwörter nicht erkannt werden; und
Klassifizierung des Berichts als "unentschiedene Infiltrate", wenn die als Infiltrate erkannten Schlüsselwörter in der Phrase erkannt werden, die Phrase aber keine Richtungswörter enthält.

9. Computerimplementiertes Verfahren nach Anspruch 1, wobei die klinische Echtzeitanwendung mindestens einen der folgenden Schritte umfasst: Anwendung des klassifizierten Berichts auf computergestützte klinische Richtlinien zur Erkennung einer Krankheit; und
Anwendung des klassifizierten Berichts zur Festlegung der Therapieempfehlungen des computergestützten klinischen Protokolls.

10. Computerimplementiertes Verfahren nach Anspruch 1, wobei die klinische Echtzeitanwendung Folgendes umfasst:
Anwenden des klassifizierten Berichts auf fortgeschrittene klinische Entscheidungshilfsrichtlinien zum Erkennen einer Krankheit, wobei das Anwenden des klassifizierten Berichts auf die fortgeschrittenen klinischen Entscheidungshilfsrichtlinien ferner umfasst: Zuweisen eines numerischen Werts zu jeder Infiltratklassifikation; und Eingeben der numerischen Werte in ein Modell zum Erkennen einer Krankheit.

11. Computerimplementiertes Verfahren nach Anspruch 1, wobei die klinische Echtzeitanwendung Folgendes umfasst:
Anwendung des Verschlusssachenberichts zur Meldung einer Krankheit oder eines klinischen Zustands in einer Gesundheitseinrichtung.

12. Computerimplementiertes Verfahren nach Anspruch 1, wobei die klinische Echtzeitanwendung Folgendes umfasst:
Anwendung des klassifizierten Berichts zur Überprüfung der Einhaltung von mindestens einem der folgenden Elemente: Krankenhauspolitik, Krankenhausqualitätsprotokoll oder Krankenhaussicherheitsprotokoll; oder Anwendung des klassifizierten Berichts zur Überwachung der Wirksamkeit der Krankenhauspolitik.

13. System zum Extrahieren von Informationen über Infiltrate aus einem Bildgebungsbericht und zum Anwenden der Infiltratinformationen des Berichts auf klinische Richtlinien und klinische Unterstützungsanwendungen, das folgendes umfasst:
ein nicht-übertragbares computerlesbares Speichermedium, das ein ausführbares Programm speichert;
einen Speicher, der eine Schlüsselwortdatenbank speichert, die infiltrat-bezogene Schlüsselwörter, Richtungswörter und Negationswörter enthält; und einen Prozessor, der das ausführbare Programm ausführt, um den Prozessor zu veranlassen, ein Verfahren nach einem der vorangehenden Ansprüche durchzuführen.

14. System nach Anspruch 13, wobei die klinische Echtzeitanwendung Folgendes umfasst: Anwendung des klassifizierten Berichts auf computergestützte klinische Richtlinien zur Erkennung einer Krankheit.

15. Nicht-übertragbares, computerlesbares Speichermedium, das einen Satz von Anweisungen enthält, die von einem Prozessor ausgeführt werden können, wobei der Satz von Anweisungen, wenn er von dem Prozessor ausgeführt wird, den Prozessor veranlasst, Vorgänge durchzuführen, die Folgendes umfassen: Empfangen eines Bildgebungsberichts;
Analysieren des Berichts nach Infiltrat-bezogenen Schlüsselwörtern; Klassifizieren des Berichts; Darstellen des klassifizierten Berichts auf einem Dashboard für die klinische Entscheidungsfindung; und Anwenden von Infiltrat-Informationen aus dem klassifizierten Bericht in einer klinischen Echtzeit-Anwendung zum Erkennen und Vorhersagen einer akuten Krankheit; **dadurch gekennzeichnet, dass** der Bericht auf der Grundlage der Infiltrat-bezogenen Schlüsselwörter und mindestens eines von Richtungswörtern, die einen Ort der Infiltrate angeben, Negationswörtern und Wörtern, die die Negationswörter aufheben, klassifiziert wird; und dass die
klinische Echtzeit-Anwendung das Anwenden des klassifizierten Berichts auf die planmäßige Anwendung von Algorithmen zur klinischen Entscheidungsfindung, CDS, umfasst;
wobei:
wenn im Bericht Infiltrat-bezogene Schlüsselwörter gefunden werden, wird ein für den Bildgebungsbericht relevanter CDS-Algorithmus angewandt; und wenn im Bericht keine Infiltrat-bezogenen Schlüsselwörter gefunden werden, wird ein CDS-Algorithmus, der Infiltrate im Bildgebungsbericht erfordert, nicht angewandt.

## Revendications

1. Procédé mis en oeuvre par ordinateur pour extraire des informations sur les infiltrats à partir d'un rapport d'imagerie, et appliquer les informations sur les infiltrats du rapport à des directives cliniques et des applications de soutien clinique, comprenant:
la réception du rapport d'imagerie;
analyser le rapport pour trouver des mots-clés relatifs aux infiltrats;
classer le rapport;
la présentation du rapport classifié sur un tableau de bord pour la prise de décision clinique; et
l'application d'informations sur les infiltrats provenant du rapport classifié dans une application clinique en temps réel pour détecter et prédire une maladie aiguë;
**caractérisé en ce que**
le rapport est classé sur la base des mots-clés liés aux infiltrats et d'au moins un des mots directionnels indiquant un emplacement des infiltrats, des mots de négation et des mots annulant les mots de négation; et **en ce que**
l'application clinique en temps réel comprend l'application du rapport classifié pour programmer l'application d'algorithmes d'aide à la décision clinique, CDS:
dans lequel:
si des mots-clés liés aux infiltrats sont trouvés dans le rapport, alors un algorithme CDS pertinent pour le rapport d'imagerie est appliqué; et
si aucun mot clé relatif aux infiltrats n'est trouvé dans le rapport, alors un algorithme CDS nécessitant des infiltrats dans le rapport d'imagerie n'est pas appliqué.

2. Procédé mis en œuvre par ordinateur selon la revendication 1, dans lequel le rapport d'imagerie comprend un rapport de radiographie thoracique.

3. Procédé mis en œuvre par ordinateur selon la revendication 1, dans lequel les mots directionnels comprennent au moins un des éléments suivants: bilatéral, unilatéral, bibasilaire, droit, gauche, les deux.

4. Procédé mis en œuvre par ordinateur selon la revendication 1, dans lequel les mots de négation comprennent au moins l'un de: non, pas, négatif, résolu, sans, et dans lequel les mots annulant les mots de négation comprennent soit: des négations multiples, soit
au moins un adjectif parmi: partiellement, légèrement, non résolu, et aucune résolution,

5. Procédé mis en œuvre par ordinateur selon la revendication 1, dans lequel les mots relatifs aux infiltrats comprennent au moins l'un de: infiltrats, consolidation, pneumonie, inflammation, infection, opacités, œdème, multilobulaire, inondation et densités.

6. Procédé mis en œuvre par ordinateur de la revendication 1, dans lequel la classification du rapport comprend en outre:
Procédé mis en œuvre par ordinateur de la revendication 1, dans lequel la classification du rapport comprend en outre
comprend:
la détection des mots-clés liés aux infiltrats dans le rapport;
rechercher les mots directionnels dans une phrase contenant les mots-clés détectés liés aux infiltrats;
la recherche des mots de négation dans la phrase;
la recherche des mots annulant les mots de négation dans la phrase;
la négation des mots-clés liés aux infiltrats et des mots directionnels, lorsque la phrase contient les mots de négation et ne contient pas les mots annulant les mots de négation; et
stocker les mots-clés liés aux infiltrats et les mots directionnels, pour chaque phrase contenant les mots de négation et ne contenant pas les mots annulant les mots de négation; et
l'attribution d'une classe au rapport en fonction de règles logiques sur une pathologie et l'emplacement des infiltrats.

7. Procédé mis en œuvre par ordinateur selon la revendication 6, dans lequel les règles logiques comprennent:
classer le rapport comme bilatéral si les mots directionnels comprennent au moins l'un des mots bilatéraux, bibasilaires ou les deux; ou les mots directionnels comprennent à la fois la gauche et la droite; et
classer le rapport comme unilatéral si les mots directionnels incluent unilatéral;
les mots directionnels incluent droite et non gauche; ou
les mots directionnels incluent gauche et non droite.

8. Procédé mis en œuvre par ordinateur selon la revendication 6, dans lequel les règles logiques comprennent:
classer le rapport comme « pas d'infiltrés », si les mots-clés liés aux infiltrats ne sont pas détectés; et
classer le rapport comme « infiltrés indécis », si les mots-clés liés aux infiltrats sont détectés dans la phrase, mais la phrase ne comprend pas de mots directionnels.

9. Procédé mis en œuvre par ordinateur selon la revendication 1, dans lequel l'application clinique en temps réel comprend au moins l'un des éléments suivants:
l'application du rapport classifié aux directives cliniques informatisées pour détecter une maladie; et l'application du rapport classifié pour déterminer des recommandations thérapeutiques de protocole clinique informatisé.

10. Procédé mis en œuvre par ordinateur selon la revendication 1, dans lequel l'application clinique en temps réel comprend:
l'application du rapport classifié à des directives avancées d'aide à la décision clinique pour détecter une maladie, dans lequel l'application du rapport classifié aux directives avancées d'aide à la décision clinique comprend en outre:
l'attribution d'une valeur numérique à chaque classification d'infiltrat; et
l'entrée des valeurs numériques dans un modèle pour détecter une maladie.

11. Procédé mis en œuvre par ordinateur selon la revendication 1, dans lequel l'application clinique en temps réel comprend:
l'application du rapport classifié pour fournir une notification d'une maladie ou d'une condition clinique dans un établissement de santé.

12. Procédé mis en œuvre par ordinateur selon la revendication 1, dans lequel l'application clinique en temps réel comprend:
l'application du rapport classifié pour vérifier la conformité avec au moins une des politiques de l'hôpital, un protocole de qualité de l'hôpital ou un protocole de sécurité de l'hôpital; ou
l'application du rapport classifié pour contrôler l'efficacité de la politique de l'hôpital.

13. Système d'extraction d'informations sur les infiltrats à partir d'un rapport d'imagerie, et d'application des informations sur les infiltrats du rapport à des directives cliniques et à des applications de soutien clinique,
comprenant:
un support de stockage lisible par ordinateur non transitoire stockant un programme exécutable;
une mémoire stockant une base de données de mots-clés, comprenant des mots-clés liés aux infiltrats, des mots directionnels et des mots de négation; et
un processeur exécutant le programme exécutable pour amener le processeur à réaliser un procédé selon l'une quelconque des revendications précédentes.

14. Système selon la revendication 13, dans lequel l'application clinique en temps réel comprend: l'application du rapport classifié aux directives cliniques informatisées pour détecter une maladie.

15. Un support de stockage non transitoire lisible par ordinateur comprenant un ensemble d'instructions exécutables par un processeur, l'ensemble d'instructions, lorsqu'il est exécuté par le processeur, amenant le processeur à effectuer des opérations, comprenant:
la réception d'un rapport d'imagerie
analyser le rapport pour trouver des mots-clés liés aux infiltrats;
classer le rapport;
la présentation du rapport classifié sur un tableau de bord pour la prise de décision clinique; et
l'application des informations sur les infiltrats provenant du rapport classifié dans une application clinique en temps réel pour détecter et prédire une maladie aiguë;
**caractérisé en ce que**
le rapport est classé sur la base des mots-clés liés aux infiltrats et d'au moins un des mots directionnels indiquant un emplacement des infiltrats, des mots de négation et des mots annulant les mots de négation; et **en ce que**
l'application clinique en temps réel comprend l'application du rapport classifié pour programmer l'application d'algorithmes d'aide à la décision clinique, CDS;
dans lequel:
si des mots-clés liés aux infiltrats sont trouvés dans le rapport, alors un algorithme CDS pertinent pour le rapport d'imagerie est appliqué; et
si aucun mot clé relatif aux infiltrats n'est trouvé dans le rapport, alors un algorithme CDS nécessitant des infiltrats du rapport d'imagerie n'est pas appliqué.
